# EUROPÄISCHE PATENTSCHRIFT

(11) **EP 1 909 718 B1**
(45) Veröffentlichungstag und Bekanntmachung des Hinweises auf die Patenterteilung: **01.05.2013**
(21) Anmeldenummer: 06753268.9
(22) Anmeldetag: 12.06.2006
(51) Int. Cl.: A61F 9/01, B23K 26/40, A61B 18/20

(54) **VERFAHREN ZUM BEARBEITEN EINES ORGANISCHEN MATERIALS**
METHOD FOR TREATING AN ORGANIC MATERIAL
PROCÉDÉ POUR TRAITER UNE MATIÈRE ORGANIQUE

(30) Priorität: 13.06.2005 DE 102005027355
(43) Veröffentlichungstag der Anmeldung: 16.04.2008
(73) Patentinhaber: Technolas Perfect Vision GmbH, 80992 München (DE)
(72) Erfinder: KORN, Georg, 14532 Kleinmachnow (DE)
(74) Vertreter: Maisch, Thomas
(86) Internationale Anmeldenummer: PCT/DE2006/001001
(87) Internationale Veröffentlichungsnummer: WO 2006/133676

(56) Entgegenhaltungen:
- DE-A1- 10 125 206
- US-B1- 6 333 485
- WEINER A M: "Femtosecond pulse shaping using spatial light modulators", REVIEW OF SCIENTIFIC INSTRUMENTS, AIP, MELVILLE, NY, US, vol. 71, no. 5, 1 May 2000 (2000-05-01), pages 1929-1960, XP012038262, ISSN: 0034-6748, DOI: 10.1063/1.1150614
- EFIMOV A ET AL, OPTICS LETTERS OPT. SOC. AMERICA USA, VOL. 23, NR. 20, PAGE(S) 1612 - 1614, ISSN 0146-9592

## Beschreibung

Die Erfindung betrifft ein Verfahren zum Bearbeiten eines organischen Materials, insbesondere eines biologischen Materials.

### Stand der Technik

Bisher bekannte Verfahren des Einsatzes von ultrakurzen Laserimpulsen zur Mikrostrukturierung von Materialien nutzen die Besonderheiten des schnellen Energieeintrages dieser Strahlung in das zu bearbeitende Material. Die Impulse sind typischerweise kürzer als die Elektron-Phonon-Kopplungsdauer des zu bearbeitenden Materials, die typischerweise unterhalb von 1 bis 2ps liegt. Das heißt, während der Einwirkung des Impulses findet nur ein sehr geringer Energietransfer an die Umgebung des lokalisierten Wechselwirkungsortes statt. Eine Zerstörung ist damit stark lokalisiert. Randschädigungen sind geringer als bei der Bearbeitung mit längeren Impulsen.

Bei Verwendung von längeren Impulsen ist während der Dauer des Impulses ein Wärmefluß in die Umgebung der bestrahlten Stelle möglich. Die räumliche Ausdehnung der zerstörten Stelle kann demzufolge deutlich größer als der Radius des einwirkenden Laserstrahls sein. Besonders drastisch beweisen das Untersuchungen mit CO₂-Lasern, die Einflußzonen bis 50µm unterhalb der bestrahlten abladierten Oberfläche aufwiesen (siehe zum Beispiel DE 101 25 206 B4).

Weiterhin ist aufgrund der hohen Intensitäten ein ausgeprägtes deterministisches Verhalten bei der Zerstörung zu beobachten. Die Ursache hierfür liegt in der zuverlässigen Generation von anfänglichen freien Ladungsträgern (Elektronen) durch Multiphotonenionisation. Die weitere Zerstörung entsteht dann in Abhängigkeit von der Impulsbreite entweder aufgrund eines Avalancheprozesses bei längeren Impulsen oder eines direkten Multiphotonenprozesses bei kürzeren Impulse mit einer Impulslänge von weniger als etwa 200fs (vgl. IEEE Vol. 31. S. 2241- 2257 (1995); Appl. Phys. Lett. Vol. 64, S. 3071-3073, (1994)). Im Vergleich dazu ist bei längeren Impulsen mit einer Impulslänge von mehr als etwa 10ps von Nanosekunden ein statistischer Charakter zu beobachten, da der Impuls nicht über die Intensität verfügt, um die notwendigen anfänglichen Ladungsträger reproduzierbar zur Verfügung zu stellen. Die nahezu freien Ladungsträger sind dann statistisch im zu bearbeitenden Material durch leicht ionisierbare Defekte verteilt. Daraus folgt der nicht deterministische Vorgang der Zerstörung für längere Impulse

Weiterhin erweist sich, dass die Zerstörungsschwelle (Maßeinheit J/cm²) bei längeren Impulsen proportional zu (τ)^{1/2} (τ - Impulsbreite) fällt. Für Impulse im Bereich oder kürzer der Elektron-Phonon-Kopplungszeit fällt die Kurve deutlich schwächer. Impulse mit Breiten einer ps oder von sub-ps benötigen ein deutlich geringere Fluence für die Zerstörung als ns-Impulse. Kurze Einzelimpulse im sub-ps-Bereich finden deswegen in der präzisen Mikrostrukturierung sowohl für die Bearbeitung von transparenten und nichttransparenten Materialien Anwendung für die Oberflächenbearbeitung als auch für die Modifikation im Volumen von Materialien Anwendung (vgl. US 5,656,186).

Aus dem Dokument DE 101 25 206 B4 ist ein Verfahren zur direkten Mikrostrukturierung von Materialien mittels ultrakurzer Einzelimpulse oder Impulsfolgen mit definiertem Energieeintrag bekannt. Bei dem bekannten Verfahren werden nacheinander zwei zeitlich geformte Laserimpulse oder Impulszüge auf die Oberfläche des zu bearbeitende Material gerichtet. Der Abstand zweier aufeinanderfolgender Impulse oder Impulszüge ist kleiner oder gleich Pikosekunden eingestellt, so daß der folgende Impuls noch in die bewirkte Änderung des - ersten Impulses im zu bearbeitenden Material trifft. Energie und Dauer des Impulses werden in Abhängigkeit vom zu bearbeitenden Material eingestellt. Das bekannte Verfahren wird genutzt, um transparente oder nichttransparente Materialien im Mikrobereich zu Strukturieren, konkrete Beispiele sind Festkörper wie Graphit und Quarzglas. Ein identisches Verfahren ist in dem Dokument EP 1 260 838 A2 beschrieben.

Der optimale Abstand der sub-ps-Impulse zur Verbesserung der Bearbeitungsresultate hängt von dem komplexen Zusammenspiel von Energielokalisation und -dissipation im bestrahlten Material ab. Die Verwendung von speziell phasenmodulierten Impulsen oder Impulsfolgen erweitert die Berabeitungsmöglichkeiten von Festkörpermaterialien durch eine mögliche Erhöhung der Intensität (eingestrahlte Energie pro Fläche) und ein gezieltes "softening" des Materials durch den ersten Impuls. Die folgenden Impulse spüren bei entsprechender Verzögerung die Änderungen des Materialzustandes durch den ersten Impuls. Das führt zu Erzeugung verbesserten Strukturen mit höherer Kantenschärfe im Inneren von Materialien und an Oberflächen durch Verringerung von Mikrorissen und Spannungen im Material.

### Die Erfindung

Aufgabe der Erfindung ist es, ein Lasersystem zum Bearbeiten eines organischen Materials, insbesondere eines biologischen Materials, anzugeben, mit dem eine hochpräzise Bearbeitung ermöglicht ist, insbesondere zum Ausführen exakter auch komplexer dreidimensionaler Schnitte oder zum Ausbilden von Kavitäten oder Formveränderungen in transparenten und nichttransparenten organischen Materialien.

Diese Aufgabe wird erfindungsgemäß durch ein Lasersystem nach dem unabhängigen Anspruch 1 gelöst. Vorteilhafte Ausgestaltungen der Erfindung sind Gegenstand von abhängigen Unteransprüchen.

Erfindungsgemäß ist ein Lasersystem zum Bearbeiten eines organischen Materials, insbesondere eines biologischen Materials, bei dem das organische Material zum definierten Energieeintrag mit Laserlicht in Form ultrakurzer Impulse bestrahlt wird, welche hinsichtlich einer jeweiligen Impulslänge und einer Impulsenergie für das organische Material eingestellt sind, so dass eine Energiedichte von etwa 100mJ/cm² bis etwa 100J/cm² gebildet ist, wobei nacheinander mindestens zwei Impuls oder Impulsfolgen auf eine Oberfläche des organischen Materials eingestrahlt werden und wobei ein zeitlicher Abstand zwischen einem vorangehenden Impuls / einer vorangehenden Impulsfolge und einem folgenden Impuls / einer folgenden Impulsfolgen kleiner oder gleich Pikosekunden ist und der folgenden Impuls / die folgende Impulsfolge noch in eine mittels des vorangehenden Impulses / der vorangehenden Impulsfolge bewirkte Änderung in dem organischen Material trifft, so dass eine dauerhafte Veränderung des organischen Materials gebildet wird.

Der zeitliche Abstand der Impulse der aufeinanderfolgenden Impulse / Impulsfolgen und die Dauer der Einzelimpulse ist demzufolge kleiner als die Elektron-Phonon-Wechselwirkungsdauer einzustellen. Nur in diesem Fall kommen die Vorteile des hier beschriebenen Verfahrens zur Verbesserung von Materialbearbeitungsresulten mittels kurzer Laserimpulse zum Tragen.

Ein wesentlicher Vorteil der Erfindung besteht darin, dass mittels der zeitlichen Formung der eingestrahlten Lichtimpulse und der gezielten zeitlichen Abfolge der Impulse / Impulsfolgen für das zu bearbeitende organische Material die Strahlenbelastung für Bereich hinter dem Bestrahlungsort entlang der Ausbreitungsrichtung des Laserstahls reduziert wird. Es ist auf diese Weise für organische Materialien ermöglicht, gezielt innerhalb des organischen Materials Veränderungen auszuführen, die nicht auf den Bereich der Oberfläche beschränkt sind, wie dies im Stand der Technik für andere Materialien der Fall ist (vgl. DE 101 25 206 B4).

Es ergibt sich weiterhin der Vorteil, dass im Vergleich zu bekannten Verfahren der Laserlichtbearbeitung die Genauigkeit der Bearbeitung erhöht werden kann, bis zu einer Genauigkeit von <1 µm, da die Zerstörung gezielt und beschränkt auf der Achse des Laserstrahls erzeugt wird. Das lässt sich durch den Abstand der Impulse und den Energieinhalt sowie die zeitliche Formung der verwendeten Impulse optimieren.

Bei dem geschaffenen Lasersystem werden eine minimale Energiedichte und Intensität der eingestrahlten Impulse / Impulsfolgen gewahrt, um typische bei der Verwendung von Femtosekunden erzielte deterministische Zerstörungen zu erzielen. Insbesondere kann mit dem beschriebenen Verfahren die mittlere Intensität im Wechselwirkungsbereich der Strahlung mit dem Medium reduziert werden. Ein kurzer intensiver fs-Impuls mit geringerer Energie generiert die notwendige Startdichte der freien Ladungsträger, ein verzögerter zweiter längerer sub-ps-Impuls strahlt in die gleiche vorbehandelte Stelle ein. Dieser zweite Impuls kann die Funktion der eigentlichen Materialbearbeitung, beispielsweise des Schneidens oder des Materialabtragens, im Innern des bearbeiteten Materials oder an seiner Oberfläche übernehmen.

Ein besonderer Vorteil bei der Verwendung des vorgeschlagenen Lasersystems zum Bearbeiten biologischer Materialien, welcher aber auch für andere organische Materialien vorteilhaft sein kann, besteht darin, dass die Strahlenbelastung hinsichtlich der Intensität und der Energiedichte der eingestrahlten Impulse / Impulsfolgen optimiert und hierdurch gering gehalten werden kann, um die Zerstörung gut zu lokalisieren und Randeffekte wie Materialspannungen (im Volumen um die zerstörte Stelle) zu reduzieren, die durch zu starke Schockwellen entstehen können. Hierdurch bleibt bei transparenten Materialien die Transparenz nach der Bearbeitung (Behandlung) besser erhalten, und das Bilden von Streuzentren im Materialien werden reduziert. Die Homogenität des bearbeiteten Materials bleibt besser erhalten.

Dieser Vorteil entfaltet seine Wirkung insbesondere bei Verwendung des Verfahrens zur Modifizierung von Hornhautgewebe zum Schneiden eines sogenannten Flaps im Rahmen eines LASIK-Verfahrens (LASIK - Laser in situ Keratomileusis) und zur intrastromalen Korrektur von Fehlsichtigkeiten mittels Erzeugung speziell geformter Kavitäten zur Korrektur von Myopie, Hyperopie oder unterschiedlicher Formen des Astigmatismus sowie bei der wellenfrontgeführten Korrektur mit Reduzierung bzw. Eliminierung der höheren Aberrationen. Nach einer solchen Behandlung sollen ein hoher Kontrast (Sehschärfe) sowie die notwendige Refraktion des Auges hergestellt sein. Es wird erwartet, dass sich bei Patienten teilweise beobachtete Photophobien (starke Lichtempfindlichkeiten) des mit fs-Impulsen behandelten Auges reduzieren oder sogar gänzlich eliminieren lassen. Diese sind zumindest in einigen Fällen offensichtlich die Folge einer Kombination von zu hoher Strahlenbelastung der Netzhaut durch den schneidenden fs-Laser und der erhöhten Streuung des behandelten Stroma nach der Operation sowie der Störung der Heilungsprozesse durch die Veränderung der Hornhaut bei Einwirkung eines intensiven fs-Impulses.

Eine wesentliche Besonderheit bei der Bestrahlung mit Femtosekunden zum Zweck der kontrollierten präzisen Zerstörung, beispielsweise einer Schnittführung oder einer Kavitätenerzeugung, ist die zeitliche Abfolge des ablaufenden Prozesses: Zu Beginn der Bestrahlung werden zunächst auf der Vorderflanke des Impulses aufgrund von Multiphotonenionisation freie Ladungsträger erzeugt. Das entstehende Plasma weist aber noch nicht die kritische Dichte auf. Deswegen kann ein Teil der angewendeten Strahlungsenergie durch den Wechselwirkungspunkt hindurchtreten. Das biologische Gewebe ist in diesem Fall transparent und die hindurchtretende Strahlung trifft im Fall einer Augenbehandlung ungehemmt auf die Netzhaut. Das kann zu Verblendungen der Photorezeptoren bis hin zur Schädigung der Netzhaut führen. Es ist also wünschenswert diese Belastungen der Netzhaut deutlich unterhalb eines kritischen Wertes (1µJ/cm²) zu senken.

Messungen haben gezeigt, dass eine Reduzierung der Impulsbreite von 3ps auf 125fs eine Verringerung der transmittierten Energie bei der Plasmabildung in Wasser um etwa 20% für Laserspotgrößen zwischen 7µm-10µm (vgl. Appl. Optics 36(22)5630-5640,1997). Das kann bei dem vorgeschlagenen Verfahren dadurch erreicht werden, dass ein erster kurzer Impuls mit geringerer Energie und hohem Kontrastverhältnis die Erzeugung der kritischen Plasmadichte übernimmt. Der Intensitätskontrast des ersten Impulses sollte etwa 10³ betragen, um die Wechselwirkung der Impulse mit dem zu bearbeitenden Material auf gezielte Art und Weise zu gestalten, so dass die Vorteile der Lasermaterialbearbeitung mit Femtosekunden zum Tragen kommen. Der zweite eigentlich bearbeitende längere Impuls oder die nachfolgenden Teile des speziell phasenmodulierten Impulses werden dann in dem erzeugten dichten Plasma vollständig absorbiert, die im Fall der Augenbehandlung auf die Netzhaut durchtretende Laserenergie ist hierdurch reduziert, und die lokale präzise gesteuerte Zerstörung des Hornhautmaterials ist gewährleistet. Der zeitliche Intensitätsverlauf des zweiten Impulses / der Impulsfolge kann beispielsweise mittels adaptiver Steuerung des Phasenverlaufs optimal gestaltet werden. Optimal zur Reduzierung der Intensitätsbelastung sowie zur Minimierung der durch das Plasma transmittierten Energie ist beispielsweise ein verlängerter abfallender Intensitätsverlauf mit zeitlicher Dauer. Die eigentlich zur Zerstörung und Ablation notwendige Energie ist am lang auslaufenden Ende des Impulses konzentriert.

Weiterhin ist wichtig, dass die Hornhautschnitte oder die Erzeugung von Kavitäten zur intrastromalen Korrektur von Fehlsichtigkeiten mit relativ kurzer Dauer ausgeführt werden, weshalb die Verwendung ein hochrepetierender Laser mit Energien im Bereich von einigen µJ, beispielsweise etwa 0.1 bis 1µJ für den ersten Impuls und etwa 1 bis 5µJ für den folgenden Impuls, bevorzugt wird, um die Behandlungszeit auf kurze Zeiten zu reduzieren, bevorzugt deutlich kürzer als eine Minute. Die eines Lasersystems mit hoher Wiederholfrequenz ist aus vergleichbaren Gründen jedoch auch für andere Anwendungen zweckmäßig.

Ein weiteres beobachtetes Phänomen ist die Bildung von sogenannten "streaks", bei denen es sich um Trübungen des Stroma im Bereich der Wechselwirkungszone handelt, für die eine laterale Ausdehnungen von etwa 500nm beobachtet wurde, die bis zu etwa 100µm in beiden Richtungen entlang der Strahlrichtung des Laserstrahls im mit fs-Impulsen behandelten Hornhautstroma sichtbar sind (siehe Heisterkamp, Appl. Phys. B74, 419-425). Dies ist Folge einer Selbstfokussierung der intensiven fs-Impulse vor dem beabsichtigten Wechselwirkungsort, da die Peakleistungen der fs-Impulse oberhalb eines kritischen Wertes liegen können. Die "streaks" können zu Trübungen in der Hornhaut führen. Eine Reduzierung der mittleren Intensität, wie sie bei dem hier vorgeschlagenen Verfahren möglich ist, sowie die Wahl eines geeigneten Fokussierungswinkels verringern diese unerwünschten Belastungseffekte oder eliminieren sie vollständig. Das führt zu einer klareren Hornhaut nach der Behandlung mit den fs-Impulsen. Es ist eine Reduzierung von Patientenfällen mit Photophobie nach fs-Behandlung zu erwarten.

Bei einer Wellenfront geführten Ablation werden mittels Erhöhung der Präzision des Schnittes sowie der verbesserten Repositionierung des Flaps nach der Behandlung mit dem hier vorgestellten Verfahren Verbesserungen hinsichtlich der Aberrationskorrektur erzielt. Bisher haben insbesondere bei der LASIK die mechanischen Verwerfungen durch den Schnitt mit dem Mikrokeratom einen starken, oft nicht reproduzierbaren Einfluß auf die entstehenden Restaberrationen des Auges nach der Behandlung. Eine Verbesserung der Genauigkeiten des fs-Laserschnitts und der erzielbaren Repositioniergenauigkeit des Flaps führt zur angestrebten mittleren quadratischen Wellenfrontdeformation in die Größenordnung unterhalb von λ/10 (RMS - "root mean square") oder λ/14, wodurch sich dem theoretischen beugungsbgrenzten Auflösungsvermögen genähert wird. Das wird, wegen der Genauigkeitserhöhung des Verfahrens, der Wellenfront geführten Ablation mit ns-UV-Impulsen eine weitere Verbesserung ermöglichen.

Auch wenn einzelne Vorteile der Erfindung vorangehend in Verbindung mit einer Bearbeitung von biologischem Gewebe am Auge erläutert wurden, entfalten die jeweiligen Vorteile ihre positiven Wirkungen auch bei anderen organischen Materialien entsprechend, beispielsweise im Zusammenhang mit Operationen im Gehörgang oder bei der Bearbeitung von Zahmaterial.

### Ausführungsbeispiele der Erfindung

Die Erfindung wird im folgenden anhand von Ausführungsbeispielen unter Bezugnahme auf Figuren einer Zeichnung näher erläutert. Hierbei zeigen:
- Fig. 1: eine schematische Darstellung eines Lasersystems zur Verwendung beim Bearbeiten eines organischen Materials mit ultrakurzen Lichtimpulsen; und
- Fig. 2: eine schematische Darstellung einer Anordnung zum Erzeugen von Impulsen unter Nutzung eines Michelson-Inteferometers zum Erzeugen einer zusätzlichen Phasenmodulation bei dem Lasersystem nach Fig. 1.

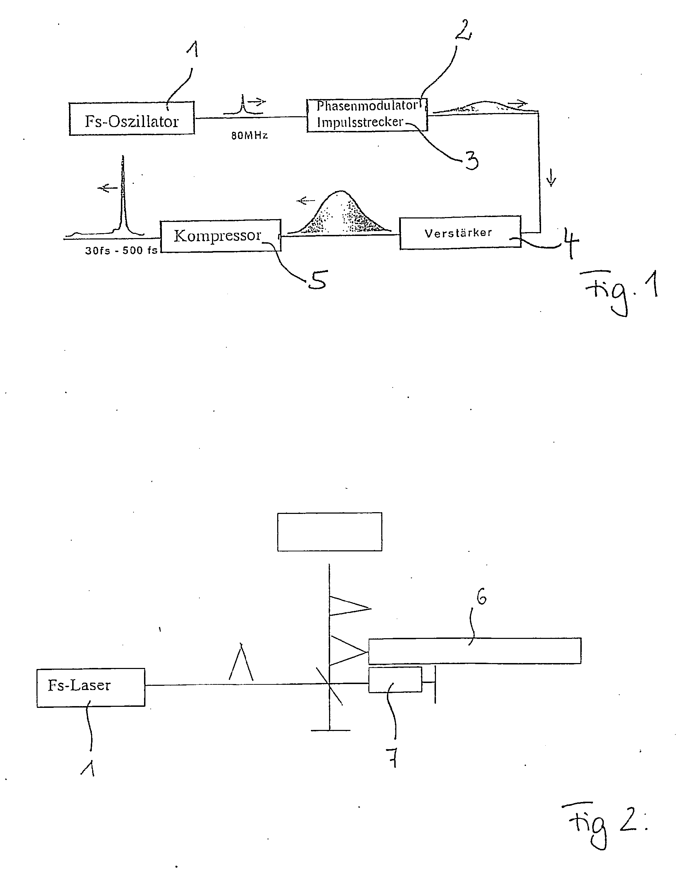

Fig. 1 zeigt eine schematische Darstellung eines CPA-Lasersystems (CPA- "chirped Impulse amplification"), mit dem ultrakurze Impulse zum Bearbeiten eines organischen Materials erzeugt werden. Die erzeugten Impulse / Impulsfolgen, die speziell phasenmoduliert sein können, können für Bearbeitung des jeweiligen organischen Materials hinsichtlich ihres zeitlichen Verlaufs und ihrer Energie eingestellt werden. Eine Modulation wird im Allgemeinen mittels Abstimmung des Oszillatorniveaus erreicht.

Von einem fs-Oszillator 1 mit einer Wiederholfrequenz von etwa 80 bis100 MHz erzeugte Impulse werden auf einen Phasenmodulator 2 gegeben. Danach läuft der so behandelte Impuls in einen Impulsstrecker 3 der einen linearen "chirp" zusätzlich zur bereits bestehenden Phasenmodulation aufprägt. Die anschließend in einem Verstärker 4 verstärkten Impulse werden danach in einem Kompressor 5 zu fs-Impulsen mit hoher Energie gewandelt. Die so erzeugten Impulse / Impulsfolgen, die aufgrund der nichtkompensierten zusätzlich aufgeprägten Phasenmodulation phasenmoduliert sind, werden dann zur Bearbeitung des organischen Materials eingesetzt, beispielsweise im Rahmen eines Schnittes für ein LASIK-Verfahren oder einer Fehlsichtigkeitskorrektur durch intrastromale Veränderung am Auge.

Es ist also eine zusätzliche / separate Phasenmodulation vorgesehen. Bei herkömmlichen CPA-Lasersystemen im Bereich über 100fs wird hauptsächlich der lineare "chirp" des Impulsstreckers 3 durch den Kompressor 5 beseitigt. Der so gebildete Impuls weist nach der Verstärkung nahezu seine ursprüngliche Impulslänge auf.

Ausgehend von dem Lasersystem nach Fig. 1 kann beispielsweise mit einer Anordnung nach Fig. 2 ein optimierter Doppelimpuls zur Bearbeitung zur Verfügung gestellt werden, der die obengenannten Kriterien für eine möglichst schonende Behandlung des organischen Materials erfüllt. Für gleiche Merkmale werden in Fig. 2 die gleichen Bezugszeichen wie in Fig. 1 verwendet.

Vom fs-Oszillator 1 kommend wird der Impuls durch einen als Michelson-Interferometer ausgebildeten Phasenmodulator 2 geschickt. In einem optischen Arm 6 steht zusätzlich ein abstimmbares dispersives Element 7 zur Verfügung, welches einem der erzeugten Impulse neben einer Verzögerung eine abstimmbare Dispersion, die hauptsächlich 2. Ordnung ist, aufprägt. Danach erfahren die beiden abstimmbar zueinander verzögerbaren Impulse eine Dehnung auf einen Wert zwischen etwa 10ps bis 500ps in dem Impulsstrecker 3.

Nach der Verstärkung ist der Kompressor 5 so eingestellt, dass nur der erste Impuls einen Wert von etwa 50 bis 500fs hinsichtlich der Impulsbreite aufweist. Der folgende Impuls kann in Abhängigkeit von der zusätzlich im Michelson-Interferometer 2 aufgeprägten Dispersion eine abstimmbare Breite zwischen etwa 100fs und 3ps bei einstellbarer zeitlicher Verzögerung zwischen den verstärkten Impulsen aufweisen. Die Optimierung des Bearbeitungsergebnisses wird mit Hilfe der Wahl der zeitlichen Verzögerung und der zusätzlich eingeführten Dispersion erzielt. Methoden der Phasenmodulation können mittels der gezielten Veränderung der Phasenverzögerung bei unterschiedlichen Wellenlängen beispielsweise in einem "zero dipersion stretcher" oder mittels akusto-optischer Methoden erzielt werden. Das Ergebnis der Materialbearbeitung kann dann mit Hilfe einer Rückkopplungsschleife mittels Computer optimal gestaltetet werden (A. M. Weiner, "Femtosecond Pulse Shaping Using Spatial Light Modulators", Rev. Sci Instrum. 71, pp. 1929-1960, 2000.)

Wesentlich ist, dass das beschriebene CPA-System den Impuls zum Zwecke der Verstärkung zeitlich im Strecker dehnt (vgl. US RE37,585). Dieser verlängerte Impuls kann dann im Verstärkersystem zu hohen Energien gebracht werden, ohne dass sich nichtlineare Effekte bei der Verstärkung bemerkbar machen. Nach der Verstärkung wird der Impuls dann wieder zu seiner Originallänge komprimiert. Im Gegensatz dazu weist in dieser Schrift der oder die Impulse eine zusätzliche Phasenmodulation zum stretcher auf, der durch den Kompressor nicht kompensiert werden kann bzw. soll und dadurch zur Ausbildung von DoppelImpulsen, Impulsfolgen oder speziell zeitlich geformten Impulsen führt, die variable Impulslängen oder Abstände aufweisen und es gestatten, den Bearbeitungsprozess in der oben beschriebenen Art und Weise. zu optimieren.

Besonders geeignet zur Ausführung des Verfahrens sind direkt diodengepumpte Femtosekundenlasersysteme, deren Zentralwellenlänge im Bereich um etwa 1µm liegt. Herauszuheben sind Yb-dotierte Materialien, die einen geringen Stoke-Verschiebung aufweisen und effizient und kostengünstig mit Dioden geringer Leistung gepumpt werden können. Die Bandbreite dieser Materialien gestattet die Erzeugung und die Verstärkung von Impulsen bis unter 100fs. Die Oszillatoren lassen sich mittels SESAMs (SESAM - "*Semiconductor Saturable Absorber Mirror*") auslegen, was die Stabilität und Zuverlässigkeit dieser Systeme wesentlich verbessert hat. Insbesondere sind die Wellenlängen um etwa 1µm gut zur Behandlung des Auges geeignet, da die Hornhaut eine gute Transparenz aufweist und die Empfindlichkeit der Photorezeptoren der Netzhaut stark reduziert ist im Vergleich zum nahen Infrarot (Ti:Saphir-Laser). Die Methoden der Phasenmodulation sind generell anwendbar für Femtosekundensysteme.

Es findet keine Erhöhung der erzielbaren Energiedichte statt. Die Intensität soll insgesamt durch Aufteilung in Mehrfach-Impulse, Impulsfolgen bzw. speziell phasenmodulierte Impulse reduziert werden, um ein möglichst minimal invasives und weniger schädigendes und sehr präzises Verfahren zur Materialbearbeitung zu erzeugen.

Die in der vorstehenden Beschreibung, den Ansprüchen und den Zeichnungen offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Lasersystem zum Bearbeiten eines organischen Materials, insbesondere eines biologischen Materials, mit einem fs-Oszillator (1), einem als Interferometer ausgebildeten Phasenmodulator (2) und einem Impulsstrecker (3), gefolgt von einem Verstärker (4) und einem Kompressor (5), wobei mit dem Lasersystem das organische Material zum definierten Energieeintrag mit Laserlicht in Form ultrakurzer Impulse bestrahlbar ist, welche hinsichtlich einer jeweiligen Impulslänge und einer Impulsenergie für das organische Material derart einstellbar sind, dass eine Energiedichte von etwa 100mJ/cm² bis etwa 100J/cm² realisierbar ist, wobei das Lasersystem derart ausgebildet ist, dass nacheinander mindestens zwei Impulse oder Impulsfolgen auf eine Oberfläche des organischen Materials einstrahlbar sind und dass ein zeitlicher Abstand zwischen einem vorangehenden Impuls / einer vorangehenden Impulsfolge und einem folgenden Impuls / einer folgenden Impulsfolge kleiner oder gleich Pikosekunden realisierbar ist und der folgende Impuls / die folgende Impulsfolge noch in eine mittels des vorangehenden Impulses / der vorangehenden Impulsfolge bewirkbare Änderung in dem organischen Material trifft, so dass eine dauerhafte Veränderung des organischen Materials realisierbar ist.

2. Lasersystem nach Anspruch 1, **dadurch gekennzeichnet, dass** das organische Material mittels einer Ablation oder einer Zerstörung bearbeitbar ist.

3. Lasersystem nach Anspruch 1 oder 2, **dadurch gekennzeichnet, dass** die Oberfläche des organischen Materials strukturierbar ist.

4. Lasersystem nach einem der vorangehenden Ansprüche, **dadurch gekennzeichnet, dass** die Oberfläche des organischen Materials schneidbar ist.

5. Lasersystem nach einem der vorangehenden Ansprüche, **dadurch gekennzeichnet, dass** ein oder mehrere Schnitte im Inneren des organischen Materials führbar sind.

6. Lasersystem nach Anspruch 4 oder 5, **dadurch gekennzeichnet, dass** ein Schnitt nach einem vordefinierten dreidimensionalen Schema führbar ist.

7. Lasersystem nach einem der vorangehenden Ansprüche, **dadurch gekennzeichnet, dass** das organische Material im Inneren strukturierbar ist.

8. Lasersystem nach einem der vorangehenden Ansprüche, **dadurch gekennzeichnet, dass** organisches Material in einem Auge bearbeitbar ist.

9. Lasersystem nach Anspruch 8, **dadurch gekennzeichnet, dass** ein LASIK-Schnitt (LASIK-Laser-Assisted *In Situ* Keratomileusis) in einer Hornhaut ausführbar ist.

10. Lasersystem nach Anspruch 9, **dadurch gekennzeichnet, dass** bei dem LASIK-Schnitt eine wellenfrontgeführte Ablation ausführbar ist.

11. Lasersystem nach Anspruch 9 oder 10, **dadurch gekennzeichnet, dass** der LASIK- Schnitt nach einem vordefinierten dreidimensionalen Schema lamellierend führbar ist.

12. Lasersystem nach einem der Ansprüche 8 bis 11, **dadurch gekennzeichnet, dass** Haupt- und Nebeninzisionen in einer Sklera und / oder einer Hornhaut zur Kataraktoperation ausführbar sind.

13. Lasersystem nach einem der Ansprüche 8 bis 12, **dadurch gekennzeichnet, dass** sklerale und / oder korneale Schnitte für die Vitrektomie (pars plana) sowie Inzisionen in einem Glaskörperraum und in einer Netzhaut ausführbar sind.

14. Lasersystem nach einem der Ansprüche 8 bis 13, **dadurch gekennzeichnet, dass** asymmetrische Schnitte in dreidimensionaler Form für die Keratoplastik ausführbar sind.

15. Lasersystem nach einem der Ansprüche 8 bis 14, **dadurch gekennzeichnet, dass** Gewebe für eine Glaukomchirurgie im Kammerwinkel (im Sinne einer Goniotomie) inzidierbar ist wird oder fistulierende Schnitte durch andere Gewebsabschnitte anlegbar sind.

16. Lasersystem nach einem der Ansprüche 8 bis 15, **dadurch gekennzeichnet, dass** Linsengewebe zur Eindämmung von Presbyopie veränderbar ist und bei der Kataraktchirurgie bei geplanter Entfernung des Gewebes lockerbar ist.

17. Lasersystem nach einem der Ansprüche 8 bis 16, **dadurch gekennzeichnet, dass** bei einer Schieloperation der Muskel schneidbar ist wird mit der Zielstellung einer Verkürzung oder Verlängerung.

18. Lasersystem nach einem der Ansprüche 8 bis 17, **dadurch gekennzeichnet, dass** verschlossene Tränenwege erweiterbar oder öffenbar sind.

19. Lasersystem nach einem der Ansprüche 8 bis 18, **dadurch gekennzeichnet, dass** Entspannungsschnitte in horizontaler, vertikaler und anderer dreidimensionaler Richtung in der Hornhaut führbar sind, um Refraktionsanomalien zu korrigieren.

20. Lasersystem nach einem der vorangehenden Ansprüche, **dadurch gekennzeichnet, dass** in dem organischen Material Kavitäten und / oder Kanäle bildbar sind.

21. Lasersystem nach einem der vorangehenden Ansprüche, **dadurch gekennzeichnet, dass** die ultrakurzen Impulse mit Hilfe eines impulsformenden Verfahrens in einem Kurzimpulslaser erzeugbar sind.

22. Lasersystem nach Anspruch 21, **dadurch gekennzeichnet, dass** ein impulsformendes, phasenmodulierendes Verfahren in einem CPA-Laser zur Verstärkung der ultrakurzen Impulse verwendbar ist.

23. Lasersystem nach einem der vorangehenden Ansprüche, **dadurch gekennzeichnet, dass** der fs-Oszillator (1) ein hochenergetischer "cavity-dumped" Oszillator ist.

24. Lasersystem nach einem der vorangehenden Ansprüche, **dadurch gekennzeichnet, dass** ein aktives Element eines Lasers aus direkt diodengepumpten Yb-dotierten Materialien wie beispielsweise Yb-Glas, KY(WO₄)₂ KGd(WO₄)₂, Sc₂O₃, CaF₂ oder Y₂O₃- Keramkimaterial in einem Oszillator und / oder einem Verstärker verwendet ist, mit Wellenlängen der Laserstrahlung im infraroten Spektralbereich von etwa 1.0 µm bis etwa 1.2µm.

25. Lasersystem nach einem der vorangehenden Ansprüche, **dadurch gekennzeichnet, dass** als ein impulserzeugender Laser ein mit seltenen Erden dotierter Faserlaser verwendet ist.

26. Lasersystem nach Anspruch 25, **dadurch gekennzeichnet, dass** ein Faserverstärker verwendet ist, welcher eine Doppelmantelstruktur aufweist.

## Claims

1. Laser system for treating an organic material, in particular a biological material, having an fs oscillator (1), a phase modulator (2) in the form of an interferometer and a pulse stretcher (3), followed by an amplifier (4) and a compressor (5), wherein the organic material can be irradiated by the laser system with laser light in the form of ultrashort pulses for the defined energy input, said pulses being adjusted in terms of a particular pulse length and a pulse energy for the organic material, such that an energy density of from approximately 100mJ/cm² to approximately *100Jlcm²* can be obtained, wherein the laser system is designed in such a manner that at least two pulses or pulse sequences can be irradiated consecutively onto a surface of the organic material, and a time interval between a preceding pulse/a preceding pulse sequence and a following pulse/a following pulse sequence that is smaller than or equal to picoseconds can be achieved, and the following pulse/the following pulse sequence hits the organic material again where a change caused by the preceding pulse or the preceding pulse sequence has taken place, so that a permanent change in the organic material can be created.

2. Laser system according to claim 1, **characterised in that** organic material is treatable by means of ablation or destruction.

3. Laser system according to claim 1 or 2, **characterised in that** the surface of the organic material can be structured.

4. Laser system according to any one of the preceding claims, **characterised in that** the surface of the organic material can be cut

5. Laser system according to any one of the preceding claims, **characterised in that** one or more incisions can be made in the interior of the organic material.

6. Laser system according to claim 4 or 5, **characterised in that** an incision can be made according to a predefined three-dimensional model.

7. Laser system according to any one of the preceding claims, **characterised in that** the interior of the organic material can be structured.

8. Laser system according to any one of the preceding claims, **characterised in that** organic material in an eye is treatable.

9. Laser system according to claim 8, **characterised in that** a LASIK incision (LASIK - Laser-Assisted In Situ Keratomileusis) can be made in a cornea.

10. Laser system according to claim 9, **characterised in that** a wavefront-guided ablation can be carried out during the LASIK incision.

11. Laser system according to claim 9 or 10, **characterised in that** the LASIK incision can be made in a lamellar fashion according to a predefined three-dimensional model.

12. Laser system according to any one of claims 8 to 11, **characterised in that** main and auxiliary incisions can be made in a sclera and/or cornea for a cataract operation.

13. Laser system according to any one of claims 8 to 12, **characterised in that** scleral and/or corneal incisions can be made for a vitrectomy (pars plana) and incisions can be made in a vitreous cavity and in a retina.

14. Laser system according to any one of claims 8 to 13, **characterised in that** asymmetrical incisions can be made in three-dimensional form for keratoplasty.

15. Laser system according to any one of claims 8 to 14, **characterised in that** tissue can be incised in the chamber angle (in the context of a goniotomy) for glaucoma surgery, or fistulising incisions can be made through other tissue sections.

16. Laser system according to any one of claims 8 to 15, **characterised in that** lens tissue can be modified for controlling presbyopia and in cataract surgery can be loosened during planned removal of tissue.

17. Laser system according to any one of claims 8 to 16, **characterised in that** the muscle can be cut during strabismus surgery with the objective of shortening or extending it.

18. Laser system according to any one of claims 8 to 17, **characterised in that** closed tear ducts can be widened or opened.

19. Laser system according to any one of claims 8 to 18, **characterised in that** loosening cuts can be made in the cornea in the horizontal, vertical and other three-dimensional directions in order to correct refraction anomalies.

20. Laser system according to any one of the preceding claims, **characterised in that** cavities and/or channels can be formed in the organic material.

21. Laser system according to any one of the preceding claims, **characterised in that** the ultrashort pulses can be generated in a short pulse laser using a pulse-shaping method.

22. Laser system according to claim 21, **characterised in that** a pulse-shaping, phase-modulating method can be used in a CPA laser in order to amplify the ultrashort pulses.

23. Laser system according to any one of the preceding claims, **characterised in that** a high-energy, "cavity-dumped" oscillator can be used.

24. Laser system according to any one of the preceding claims, **characterised in that** an active element of a laser consisting of directly diode-pumped Yb-doped materials such as Yb glass, KY(WO₄)₂, KGd(WO₄)₂, Sc₂O₃, CaF₂ or Y₂O₃ ceramic material is used in an oscillator and/or an amplifier, with laser beam wavelengths in the infrared spectral range of around 1.0 µm to around 1.2 µm.

25. Laser system according to any one of the preceding claims, **characterised in that** a fibre laser doped with rare earth metals is used as a pulse-generating laser.

26. Laser system according to claim 25, **characterised in that** a fibre amplifier is used, which has a double sheath structure.

## Revendications

1. Système laser destiné à traiter un matériau organique, en particulier un matériau biologique, comprenant un oscillateur fs (1), un modulateur de phase (2) réalisé comme interféromètre et un dispositif d'étalement des impulsions (3), suivi par un amplificateur (4) et un compresseur (5), étant entendu qu'avec le système laser, le matériau organique peut être exposé, en vue d'un intrant énergétique défini, à un faisceau laser sous la forme d'impulsions ultracourtes qui peuvent être réglées au niveau d'une longueur d'impulsion respective et d'une énergie d'impulsion pour le matériau organique de telle sorte qu'une densité énergétique d'environ 100 mJ/cm² à environ 100 J/cm² peut être réalisée, étant entendu que le système laser est réalisé de telle sorte qu'au moins deux impulsions ou successions d'impulsions peuvent être projetées successivement sur une surface du matériau organique et qu'un intervalle de temps entre une première impulsion / une première succession d'impulsions et une impulsion suivante / une succession d'impulsions suivante peut être réalisé dans un ordre inférieur ou égal à la picoseconde et l'impulsion suivante / la succession d'impulsions suivante atteint encore le matériau organique pendant une modification pouvant être induite par la première impulsion / la première succession d'impulsions de telle sorte qu'un changement durable du matériau organique peut être réalisé.

2. Système laser selon la revendication 1, **caractérisé en ce que** le matériau organique peut être traité au moyen d'une ablation ou d'une destruction.

3. Système laser selon la revendication 1 ou 2, **caractérisé en ce que** la surface du matériau organique peut être structurée.

4. Système laser selon l'une des revendications précédentes, **caractérisé en ce que** la surface du matériau organique peut être coupée.

5. Système laser selon l'une des revendications précédentes, **caractérisé en ce qu'**une ou plusieurs découpes peuvent être réalisées à l'intérieur du matériau organique.

6. Système laser selon la revendication 4 ou 5, **caractérisé en ce qu'**une découpe peut être réalisée selon un schéma tridimensionnel prédéfini.

7. Système laser selon l'une des revendications précédentes, **caractérisé en ce que** le matériau organique peut être structuré à l'intérieur.

8. Système laser selon l'une des revendications précédentes, **caractérisé en ce que** le matériau organique peut être traité dans un oeil.

9. Système laser selon la revendication 8, **caractérisé en ce qu'**une découpe LASIK (laser-assisted in situ keratomileusis) peut être exécutée dans une cornée.

10. Système laser selon la revendication 9, **caractérisé en ce que** dans la découpe LASIK, une ablation guidée par front d'onde peut être exécutée.

11. Système laser selon la revendication 9 ou 10, **caractérisé en ce que** la découpe LASIK peut être exécutée par lamelles selon un schéma tridimensionnel prédéfini.

12. Système laser selon l'une des revendications 8 à 11, **caractérisé en ce que** des incisions principales et secondaires peuvent être exécutées dans une sclère et/ou une cornée aux fins d'une opération de la cataracte.

13. Système laser selon l'une des revendications 8 à 12, **caractérisé en ce que** des découpes sclérales et/ou cornéennes pour la vitrectomie (pars plana) ainsi que des incisions dans une zone du corps vitré et dans une rétine peuvent être exécutées.

14. Système laser selon l'une des revendications 8 à 13, **caractérisé en ce que** des découpes asymétriques peuvent être exécutées dans une forme tridimensionnelle pour la kératoplastie.

15. Système laser selon l'une des revendications 8 à 14, **caractérisé en ce qu'**un tissu peut être incisé pour une chirurgie du glaucome dans l'angle iridocornéen (dans le sens d'une goniotomie) ou des découpes formant de fistules peuvent être réalisées à travers d'autres sections d'un tissu.

16. Système laser selon l'une des revendications 8 à 15, **caractérisé en ce que** le tissu d'un cristallin peut être modifié aux fins de la limitation de la presbytie et assoupli lors d'une chirurgie de la cataracte lorsqu'il est prévu d'enlever le tissu.

17. Système laser selon l'une des revendications 8 à 16, **caractérisé en ce que** lors d'une opération du strabisme, le muscle peut être coupé dans le but d'obtenir un raccourcissement ou un allongement.

18. Système laser selon l'une des revendications 8 à 17, **caractérisé en ce que** des conduits lacrymaux obturés peuvent être élargis ou dégagés.

19. Système laser selon l'une des revendications 8 à 18, **caractérisé en ce que** des coupes de détente peuvent être réalisées dans une direction horizontale ou verticale ou une autre direction tridimensionnelle afin de corriger des anomalies de la réfraction.

20. Système laser selon l'une des revendications précédentes, **caractérisé en ce que** des cavités et/ou des canaux peuvent être formés dans le matériau organique.

21. Système laser selon l'une des revendications précédentes, **caractérisé en ce que** les impulsions ultracourtes peuvent être produites par le biais d'un procédé de mise en forme d'impulsions dans un laser à impulsions courtes.

22. Système laser selon la revendication 21, **caractérisé en ce qu'**un procédé de mise en forme d'impulsions à modulation de phase dans un laser CPA peut être utilisé aux fins de l'amplification des impulsions ultracourtes.

23. Système laser selon l'une des revendications précédentes, **caractérisé en ce que** l'oscillateur fs (1) est un oscillateur « cavity-dumped » à haute énergie.

24. Système laser selon l'une des revendications précédentes, **caractérisé en ce qu'**un élément actif d'un laser provenant de matériaux chargés en Yb extraits directement au moyen de diodes, comme par exemple le verre à Yb, le KY(WO₄)₂, le KGd(WO₄)₂, le Sc₂O₃, le CaF₂ ou un matériau céramique à Y₂O₃, est utilisé dans un oscillateur et/ou dans un amplificateur avec des longueurs d'ondes du rayon laser dans la zone spectrale infrarouge d'environ 1,0 µm à environ 1,2 µm.

25. Système laser selon l'une des revendications précédentes, **caractérisé en ce qu'**un laser à fibres chargé de terres rares est utilisé en tant que laser produisant des impulsions.

26. Système laser selon la revendication 25, **caractérisé en ce qu'**un amplificateur à fibres est utilisé, lequel présente une structure à double enveloppe.
